# EUROPÄISCHE PATENTSCHRIFT

(11) **EP 3 582 713 B1**
(45) Veröffentlichungstag und Bekanntmachung des Hinweises auf die Patenterteilung: **18.08.2021**
(21) Anmeldenummer: 18714680.8
(22) Anmeldetag: 14.03.2018
(51) Int. Cl.: A61B 90/00, G01D 11/30, B25B 5/10, A61B 90/50, A61B 34/20

(54) **MEDIZINISCHE BEFESTIGUNGSEINRICHTUNG UND MEDIZINISCHE VORRICHTUNG**
MEDICAL FASTENING DEVICE AND MEDICAL APPARATUS
APPAREIL MÉDICAL DE FIXATION ET DISPOSITIF MÉDICAL

(30) Priorität: 31.03.2017 DE 102017003184; 15.09.2017 DE 102017008655
(43) Veröffentlichungstag der Anmeldung: 25.12.2019
(73) Patentinhaber: Joimax GmbH, 76227 Karlsruhe (DE)
(72) Erfinder: RIES, Wolfgang, 76351 Linkenheim (DE); STEEGMÜLLER, Rainer, 70839 Gerlingen (DE); TELSEMEYER, Sascha, 76185 Karlsruhe (DE); THÖLKING, Johannes, 68199 Mannheim (DE)
(74) Vertreter: Lichti - Patentanwälte Partnerschaft mbB
(86) Internationale Anmeldenummer: PCT/EP2018/000098
(87) Internationale Veröffentlichungsnummer: WO 2018/177585

(56) Entgegenhaltungen:
- WO-A2-2010/054836
- WO-A2-2010/054836
- DE-A1-102006 009 115
- DE-A1-102006 009 115
- DE-B3-102013 211 055
- DE-U1- 9 301 708
- US-A- 6 089 111
- US-A- 6 089 111

## Beschreibung

Die Erfindung betrifft eine medizinische Befestigungseinrichtung für einen Sensor gemäß der Gattung des Anspruchs 1.

Mit elektromagnetischem Sensor wird ein Sensor, wie Metalldrahtspulen, bezeichnet, der ein elektromagnetisches Feld, insbesondere ein inhomogenes, wie das eines elektromagnetischen Navigationssystems, nach Feldstärke und nach Ausrichtung erfasst.

Minimal-invasive Operationen werden heute bereits mittels navigationsgestützter Operationsverfahren durchgeführt. Es werden hierzu unterschiedliche Navigationssysteme eingesetzt. Es kommen aktive und passive Systeme zum Einsatz. Bei aktiven Systemen ist ein in den Körper eines Patienten eingebrachtes Teil, wie ein Instrument oder chirurgisches Werkzeug mit einem Sender versehen, über den sich extern die Position des Instruments oder Werkzeugs, insbesondere des distalen, am Eingriffsort befindlichen Endes bestimmen lässt. Bei passiven Systemen wird ein Feld erzeugt, welches über einen Sensor erfasst wird, wodurch wiederum die Position und Ausrichtung des Instruments oder chirurgischen Werkzeugs, insbesondere dessen distales Ende, direkt oder indirekt erfasst werden können. Direkte Erfassung des distalen Endes eines chirurgischen Instruments beinhaltet die Anordnung des Sensors am distalen Ende des Instruments selbst; indirekte Erfassung beinhaltet die feste starre Anbringung des Sensors in einer definierten Stelle, insbesondere Axialposition, an dem chirurgischen Instrument. Aufgrund des gemessenen Sensorsignals können Rückschlüsse auf die Position und gegebenenfalls Ausrichtung des distalen Endes gezogen werden. Bei der passiven Navigation hat sich insbesondere die elektromagnetische Navigation bewährt, bei der ein elektromagnetisches Feld extern um den Operationsbereich, beispielsweise durch einen Erzeuger eines elektromagnetischen Feldes in einem Kissen, auf dem der Patient liegt, erzeugt wird. Im chirurgischen Instrument eingebaute spulenartige Sensoren ermöglichen die Ortung der Instrumente, woraufhin eine Darstellung in CT- oder MRT-Bildern erfolgen kann. Dieses Verfahren beinhaltet keine Strahlenbelastung und reduziert insgesamt so die Strahlenbelastung, auch durch einen reduzierten Röntgeneinsatz. Es wird weder die Bildqualität beeinträchtigt, noch können Sensoren, da es eben keine optischen Sensoren sind, verdeckt werden. Die Bewegungsfreiheit des Operateurs wird nicht eingeschränkt, wie es bei optischen Systemen der Fall ist. Die Arbeit des Operateurs wird wesentlich erleichtert.

Die feste Integration von Sensoren in chirurgische Instrumente würde diese verteuern und darüber hinaus Probleme hinsichtlich der Sterilisation bedingen. Es werden daher Sensorhalter vorgesehen, die mit in ihnen integrierten Sensoren austauschbar an chirurgischen Instrumenten befestigt werden können und so auch bei unterschiedlichsten Instrumenten eingesetzt werden können. Das Anbringen des Halters und damit der Sensoren ist allerdings nicht trivial. Es wurde schon versucht, eine vollumfänglich geschlossene, den Sensor tragende Schelle an einem Instrument festzulegen. Dabei muss die Schelle über die Instrumentenspitze auf das jeweilige Instrument bis zu dem Befestigungsort aufgeschoben und dort fixiert werden. Nachteilig ist diese operative Handhabung mit verschmutzten Instrumenten. Gewebereste und Flüssigkeiten an der Instrumentenspitze können bei jedem Instrumentenwechsel beim Überziehen des Halters über die Spitze eines Instruments an das folgende Instrument übertragen werden.

Die DE 10 2006 009 115 A1 betrifft eine Klemmvorrichtung mit einem Klemmbügel und einem Klemmfuß zum Befestigen und Justieren eines optoelektronischen Sensors, insbesondere eines Lichtgitters, an einer Befestigungsstange. Dazu ist vorgesehen, dass eine Klemmschraube, die den Sensor und den Klemmfuß durchdringt, in ein Gewinde an der den Klemmfuß zugewandten Endseite des Klemmbügels eingreift. Dadurch wird der Klemmbügel, der die Befestigungsstange auf der dem Klemmfuß gegenüberliegenden Seite zumindest geringfügig mehr als mittig hintergreift, mit der Klemmschraube in den Klemmfuß eingezogen, so dass der Klemmbügel den Sensor zusammenwirkend mit dem Klemmfuß an der Befestigungsstange kraftschlüssig verbindet.

Die US608911 A zeigt eine Vorrichtung zum Anbringen eines Sensors an einem Fluiddruckzylinder mit einem proximalen Ende, das an einer Zugstange des Zylinders angebracht ist. Ein Spitzenabschnitt liegt an einem Zylinderrohr an, um die Position der Vorrichtung festzulegen. Das proximale Ende weist eine erste Befestigungsnut auf, in die die Zugstange eingepasst ist; und erste Stellschrauben zum Befestigen der montierten Zugstange. In dem Spitzenabschnitt ist eine zweite Befestigungsnut ausgebildet, in der der Sensor so montiert werden kann, dass seine Position eingestellt werden kann.

Die WO 2010/054836 A2 zeigt eine Klemmhalterung mit mindestens zwei Klemmbacken, wobei mindestens eine der Klemmbacken beweglich angeordnet ist, zwei profilierte Seiten aufweist, wobei die Profile der Seiten voneinander unterscheiden.

Der Erfindung liegt die Aufgabe zugrunde, eine Einrichtung zum Befestigen eines elektromagnetischen Sensors an einem medizinischen Instrument, sowie eine umfassende Sensorvorrichtung aus einem chirurgischen Instrument, einem elektromagnetischen Sensor und einer Befestigungseinrichtung zu schaffen, bei denen die genannten Nachteile vermieden und insbesondere ein einfaches Anbringen und Lösen der Verbindung zwischen der Befestigungseinrichtung und dem chirurgischen Instrument möglich sind, um so die Handhabung zu verbessern und optimal zu gestalten.

Erfindungsgemäß wird die genannte Aufgabe primär mit einer gattungsgemäßen medizinischen Befestigungseinrichtung gelöst, die die Merkmale des Anspruchs 1 aufweist.

Weiterhin wird erfindungsgemäß die Aufgabe gelöst durch eine medizinische Vorrichtung mit einer derartigen Befestigungseinrichtung, wobei die medizinische Vorrichtung weiter dadurch gekennzeichnet ist, dass das Instrument in den Aufnahmebereich des Gegenstücks einführbar ist, wobei die Befestigungseinrichtung mit dem Instrument klemmend lösbar verbunden ist und wobei der Sensor in der Sensoraufnahme angeordnet ist, wobei insbesondere das Instrument mit einer Umfangsnut versehen ist und dass die Breite des Schlittens der axialen Länge der Umfangsnut des Instruments entspricht.

Die Erfindung geht dabei von der Grundüberlegung aus, dass für eine zuverlässige Positionsbestimmung des Instruments eine dauerhaft sichere Relativposition des Sensors zum Instrument gewährleistet sein muss. Mittels der erfindungsgemäßen Befestigungseinrichtung kann der Sensor besonders einfach und schnell an dem medizinischen Instrument angebracht werden. Hierbei werden Fehlerquellen bei der Justage, insbesondere im Rahmen eines chirurgischen Eingriffs, vermieden. Darüber hinaus ist die klemmende Verbindung der Befestigungseinrichtung mit dem Instrument jederzeit einfach lösbar, so dass die Befestigungseinrichtung bei mehreren Instrumenten wiederverwertbar eingesetzt werden kann. Im Vergleich gegenüber der unmittelbaren Befestigung eines Sensors am Instrument besitzt der Benutzer mehr Freiheiten bei der Verwendung des Sensors, da die Einrichtung bei verschiedenen Instrumenten einsetzbar ist.

Der Durchbruch des Gegenstücks kann als Gewindebohrung ausgestaltet sein, wobei das Anpressstück in der Gewindebohrung mittels einer Schraubbewegung in Richtung des Gegenstücks zu bewegbar ist.

Bevorzugt ist vorgesehen, dass die Vertiefung erste, gegenüber liegende, gerade, winklig zueinander verlaufende Abschnitte und einen zweiten, gekrümmten Endabschnitt, der die ersten Abschnitte verbindet, aufweist. Hierdurch ist eine besonders einfache, zentrierte und wiederholbare Positionierung des Instruments an der Befestigungseinrichtung gegeben. Aufgrund der sicheren Positionierung des Instruments an der Befestigungseinrichtung ist die Bestimmung der Position des Instruments unanfälliger für Fehler. Alternativ dazu kann die Vertiefung im Querschnitt ausschließlich kreissegmentförmig ausgestaltet sein, wobei die Form der Vertiefung insbesondere Krümmungsradien im Bereich von 5 mm bis 7,3 mm aufweist.

Das Anpressstück kann eine Mantelfläche mit einer Umfangsrändelung aufweisen, um dem Benutzer eine einfache Handhabung zu ermöglichen.

Das Gegenstück kann Komponenten aus Kunststoff oder Metall aufweisen, insbesondere daraus gefertigt sein, wobei die Vorteile von Kunststoff in der einfachen und schnellen Fertigung liegen. Demgegenüber bietet Metall Vorteile hinsichtlich Robustheit und Materialverschleiß.

In einer bevorzugten Ausgestaltung der Erfindung kann das Anpressstück als einstückige Schraube ausgestaltet sein, was eine besonders einfache Ausgestaltung darstellt. Vorzugsweise ist das Gegenstück als einstückiger, umfangsseitig teilweise offener Bügel ausgestaltet mit zwei Wangen, die einander zugewandte Stirnseiten ausbilden und einen endlichen Abstand zueinander aufweisen. Auf diese Weise kann das Instrument seitlich durch den Zwischenraum zwischen den zwei Wangen in den freien Aufnahmebereich eingeführt werden und lässt sich somit einfach befestigen. Dabei kann der Abstand der Wangen kleiner sein als der Durchmesser des Aufnahmebereichs des Gegenstücks, um das Instrument sicher im Aufnahmebereich zu positionieren. Eine Kontaminationsgefahr durch Kontakt der Einrichtung mit einem verunreinigten distalen Ende eines Instruments ist dadurch vermieden.

In vorteilhafter Weiterbildung der Erfindung kann der Schlitten des Anpressstücks mit dem Schraubteil mittels einer Steckverbindung verbunden sein. Durch die mehrteilige Ausgestaltung des Anpressstücks mittels eines Schlittens und eines Schraubteils kann der Schlitten beispielsweise hinsichtlich der Positionierbarkeit des Instruments optimiert werden, während das Schraubteil hinsichtlich einer für den Benutzer einfachen Handhabung ausgestaltet werden kann. Die Verbindung zwischen Schlitten und Schraubteil kann insbesondere lösbar ausgestaltet sein, wodurch eine oder beide Komponenten wiederverwertbar sind. Alternativ kann der Schlitten mit dem Schraubteil fest verbunden sein.

Durch die drehbare Lagerung des Schlittens am Schraubteil können Rotationsbewegungen des Schlittens gegenüber dem Schraubteilaufgenommen werden, ohne dass das Schraubteil mechanisch beansprucht wird.

Das Schraubteil kann an einer dem Schlitten zugewandten Stirnseite einen Zapfen ausweisen, der in eine Ausnehmung des Schlittens in Eingriff bringbar ist. Dadurch ist das Schraubteil besonders einfach und schnell am Schlitten befestigbar. Dabei kann die Ausnehmung des Schlittens hinsichtlich dessen Querschnitts zentriert angeordnet sein, um eine eindeutige Befestigungsposition festzulegen. Die Ausnehmung des Schlittens kann als Durchbruch ausgestaltet sein.

Durch die drehbare Lagerung des Schlittens am Schraubteil können Rotationsbewegungen des Schlittens gegenüber dem Schraubteilaufgenommen werden, ohne dass das Schraubteil mechanisch beansprucht wird.

Das Schraubteil kann an einer dem Schlitten zugewandten Stirnseite einen Zapfen ausweisen, der in eine Ausnehmung des Schlittens in Eingriff bringbar ist. Dadurch ist das Schraubteil besonders einfach und schnell am Schlitten befestigbar. Dabei kann die Ausnehmung des Schlittens hinsichtlich dessen Querschnitts zentriert angeordnet sein, um eine eindeutige Befestigungsposition festzulegen. Die Ausnehmung des Schlittens kann als Durchbruch ausgestaltet sein.

In besonders vorteilhafter Ausgestaltung weist der Zapfen einen dem Schlitten zugewandten Ansatz auf, dessen Querschnitt größer ist als der Querschnitt des Zapfens, wobei der Schlitten an seiner, dem Schraubteil zugewandten Stirnseite eine Bohrung aufweist, deren Durchmesser kleiner ist als der Durchmesser der Ausnehmung, so dass zwischen der Bohrung und der Ausnehmung eine Hinterschneidung ausgebildet ist und der Ansatz die Hinterschneidung hintergreift. Auf diese Weise ist eine besonders einfache und zuverlässige Steckverbindung ausgestaltet, die durch unbeabsichtigte Bewegungen nicht aus Versehen lösbar ist.

Der Zapfen des Schraubteils kann innerhalb der Ausnehmung des Schlittens derart radial beabstandet zur Innenwandung der Ausnehmung angeordnet sein, dass zwischen dem Zapfen und der Ausnehmung ein radiales Spiel ausgebildet ist, was dann besonders sinnvoll ist, wenn der Schlitten ohne mechanische Beanspruchung Kippbewegungen aufnehmen soll. Zu diesem Zweck kann der Ansatz derart axial beabstandet zur Hinterschneidung angeordnet sein, dass zwischen dem Ansatz und der Hinterschneidung ein axiales Spiel ausgebildet ist.

Bevorzugt weist der Schlitten an seiner der Vertiefung des Gegenstücks zugewandten Stirnseite eine im Schnitt kreissegmentförmige Nut auf, so dass das Instrument zwischen der Nut und der Vertiefung des Gegenstücks eingespannt werden kann. Insbesondere ergibt sich der Vorteil, dass das Instrument flächig sowohl an der Nut als auch an der Vertiefung aufliegt und dadurch mechanisch besonders stabil gehalten ist. Vorzugsweise kann die Nut als Längsnut ausgestaltet sein und sich insbesondere über die gesamte Länge des Schlittens erstrecken.

Um die Steckverbindung zwischen dem Schlitten und dem Schraubteil zu vereinfachen, kann der Schlitten an mindestens einer seiner Mantelflächen mindestens jeweils einen Schlitz aufweisen.

Das Gegenstück kann einen Stecker und ein mit dem Stecker formschlüssig verbundenes Halteteil aufweisen. Insbesondere kann der Stecker mit dem Halteteil lösbar verbunden sein, so dass eine beziehungsweise beide Komponenten wiederverwertbar sind.

Vorzugsweise ist der Stecker mit dem Halteteil mittels einer Rast- und/oder einer Schnappverbindung verbunden. Hierzu kann insbesondere der Stecker an seiner dem Halteteil zugewandten Stirnseite mindestens zwei sich in axialer Richtung erstreckende Rasthaken mit jeweils einem Schenkel und einem hakenartigen Vorsprung aufweisen, die mit jeweils einer Ausnehmung des Halteteils in Eingriff bringbar sind. Vorzugsweise weist der Stecker zwei, drei beziehungsweise vier Rasthaken auf, die paarweise in gleichem Abstand zueinander an der Stirnseite des Steckers angeordnet sind. Besonders vorzugsweise sind die Rasthaken mittels manueller Betätigung lösbar.

In einer weiteren Ausgestaltung kann das Halteteil mindestens einen betätigbaren Vorsprung aufweisen zum Lösen der Verbindung zwischen dem Stecker und dem Halteteil. Dies ist insbesondere bei der Verwendung mehrerer Rasthaken sinnvoll, um die Verbindung zwischen dem Stecker und dem Halteteil schnell und einfach lösen zu können.

Um eine besonders einfache Handhabung durch den Benutzer zu ermöglichen, weist der Stecker an seinem dem Halteteil bzw. den Schenkeln abgewandten Ende ein zylindrisches Endstück mit einer Umfangsrändelung auf. Insbesondere in Verbindung mit der Rändelung des Schraubteils kann die Befestigungseinrichtung besonders einfach von einem Benutzer gegriffen und verschraubt werden.

Vorzugweise ist die Sensoraufnahme im Gegenstück, insbesondere in dessen Stecker, angeordnet. Die Einrichtung ist vorzugsweise aus Einweg-Komponenten gebildet. Auch kann die Sensoraufnahme im Halteteil vorgesehen sein. Dadurch kann der Sensor auch bei einem eingespannten Instrument ausgetauscht werden, ohne dass die Verbindung zwischen dem Instrument und der Befestigungseinrichtung gelöst werden muss.

In besonders vorteilhafter Ausgestaltung ist die Sensoraufnahme kreuzförmig oder V-förmig ausgestaltet, wobei mindestens eine mit dem Sensor verbundene Sensorleitung aus der Mantelfläche des Steckers beziehungsweise aus der Stirnseite des Steckers heraus münden kann. Die Sensoraufnahme kann mit einer Abdeckung versehen sein, die den Sensor vor äußeren Einflüssen zuverlässig schützt. Die Sensoraufnahme kann verschließbar ausgestaltet sein, wobei der Verschluss mit der Befestigungseinrichtung verschweißt oder verklebt sein kann. Insbesondere hat sich von Vorteil erwiesen, die Sensoraufnahme als kreuzförmige oder V-förmige Fräsung auszugestalten.

Weiterentwicklungen können vorsehen, dass die Einrichtung einen elektromagnetischen Sensor aufweist, der als mindestens zwei unter endlichem Winkel zueinander angeordnete, spiralförmig gewendelte Spulen ausgestaltet ist.

Höchst vorzugsweise ist der Sensor als X-förmig oder v-förmig angeordnete, spiralförmig gewickelte Spulen ausgestaltet, um beispielsweise in einem extern angelegten elektromagnetischen Feld die Position und Orientierung des Spannelements/Befestigungseinrichtung, einschließlich der Position und Orientierung des Sensors, exakt bestimmen zu können.

Für eine besonders zuverlässige Positionierung des Instruments kann das Instrument mit einer Umfangsnut versehen sein und die Breite des Schlittens der axialen Länge der Umfangsnut des Instruments entsprechen. Auf diese Weise wird ein axiales Verrutschen des Instruments entlang seiner Längsrichtung unterbunden.

In einer Ausgestaltung liegt das Instrument im Bereich der Umfangsnut flächig auf dem zweiten Endabschnitt der Vertiefung des Halteteils und/oder auf den beiden winklig zueinander verlaufenden Abschnitten und/oder auf der Nut des Schlittens auf, so dass das Instrument mechanisch reproduzierbar an der Befestigungseinrichtung angebracht werden kann.

Weitere Vorteile und Merkmale der Erfindung ergeben sich aus den Ansprüchen und der nachfolgenden Beschreibung, in der Ausführungsbeispiele der Erfindung unter Bezugnahme auf die Zeichnungen im Einzelnen erläutert sind. Dabei zeigen:
- Fig. 1: ein Spannelement einer medizinischen Befestigungseinrichtung in perspektivischer Darstellung;
- Fig. 2: einen Schlitten des Anpressstücks aus Fig. 1;
- Fig. 3: ein Schraubteil des Anpressstücks aus Fig. 1;
- Fig. 4: das Schraubteil aus Fig. 3 in einer Seitenansicht;
- Fig. 5: ein Halteteil des Gegenstücks in einer perspektivischen Ansicht;
- Fig. 6: einen Stecker des Gegenstücks in einer Seitenansicht;
- Fig. 7: das erfindungsgemäße Spannelement in einer ersten Ausgestaltung in einer Seitenansicht;
- Fig. 8: das Spannelement aus Fig. 7 in einem schematischen Querschnitt;
- Fig. 9: das erfindungsgemäße Spannelement in einer zweiten Ausgestaltung;
- Fig. 10: das Spannelement aus Fig. 9 in einem schematischen Querschnitt;
- Fig. 11: das Halteteil in einer weiteren Ausgestaltung;
- Fig. 12: das Halteteil aus Fig. 11 mit dem Anpressstück in einer schematischen Seitenansicht;
- Fig. 13: eine erfindungsgemäße medizinische Vorrichtung mit einer Befestigungseinrichtung und einem medizinischen Instrument in einer Seitenansicht;
- Fig. 14: die medizinische Vorrichtung aus Fig. 13 in einer perspektivischen Ansicht von schräg oben;
- Fig. 15: eine perspektivische Darstellung einer weiteren Ausgestaltung der erfindungsgemäßen Einrichtung;
- Fig. 16: einen Schnitt durch die Darstellung der Fig. 15;
- Fig. 17: eine Aufsicht der Einrichtung gemäß der Fig. 15;
- Fig. 18: eine Explosionsdarstellung durch die Einrichtung gemäß Fig. 15 mit abgenommenem Umfangsmantel bzw. Abdeckung und hierdurch sichtbaren Sensor;
- Fig. 19: eine perspektivische Darstellung einer zweiten Sensorvorrichtung mit der Einrichtung gemäß den Fig. 15 bis 18;
- Fig. 20: eine Seitenansicht der Vorrichtung aus Fig. 19;
- Fig. 21: eine Seitenansicht, die gegenüber der Fig. 20 um 180° um die Längsachse verdreht ist, und
- Fig. 22: einen Längsschnitt durch die Vorrichtung der Fig. 19 bis 21.

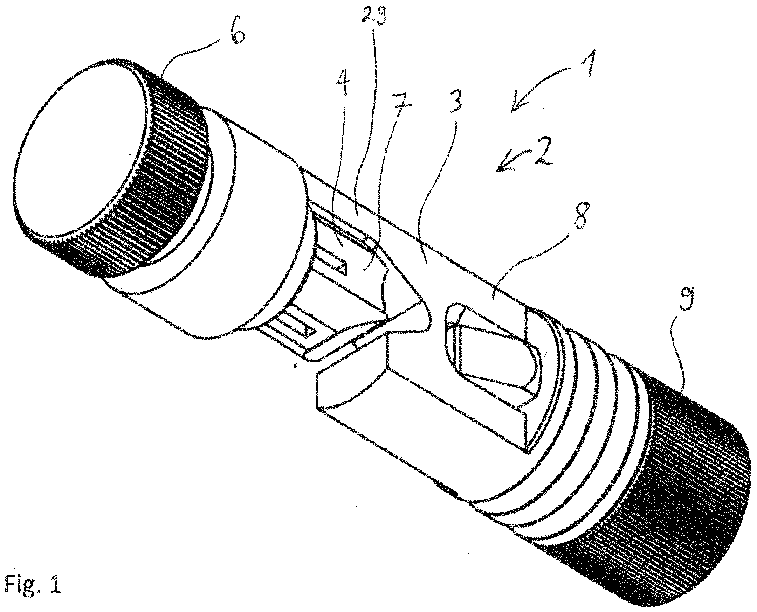

Fig. 1 zeigt eine medizinische Befestigungseinrichtung 1 in einer perspektivischen Ansicht in der Ausgestaltung als Spannelement 2, das ein auf der linken Seite gezeigtes Anpressstück 4 und ein auf der rechten Seite angeordnetes Gegenstück 3 aufweist. Das Anpressstück 4 ist mit dem Gegenstück 3 über einen Haltebereich 29 lösbar verbunden und relativ zu diesem entlang des Haltebereichs 29 axial beweglich. Auf diese Weise ist ein zwischen dem Gegenstück 3 und dem Anpressstück 4 eingebrachtes medizinisches Instrument 5 im Spannelement 2 klemmbar, was weiter unten detailliert beschrieben ist. In der in Fig. 1 gezeigten Ausführungsform weist das Anpressstück 4 ein Schraubteil 6 und einen Schlitten 7 und das Gegenstück 3 ein Halteteil 8 und einen Stecker 9 auf, die im Folgenden detailliert beschrieben werden.

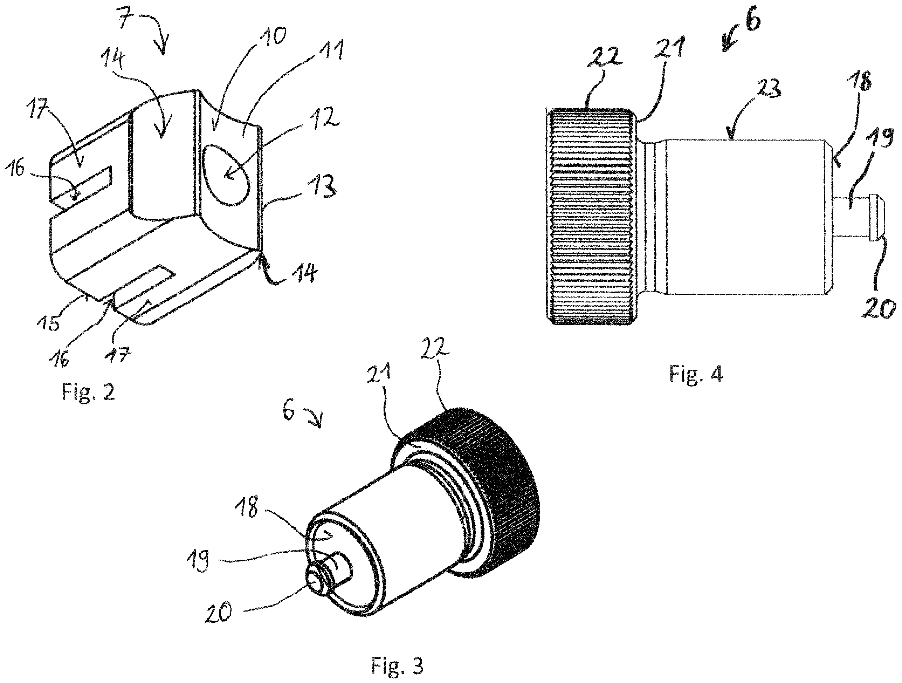

Der Schlitten 7 des Anpressstücks 4 ist in Fig. 2 in einer perspektivischen Darstellung gezeigt. Auf einer dem Gegenstück 3 zugewandten Stirnseite 10 ist eine kreisbogenförmig Nut 11 eingearbeitet, die sich über die gesamte Breite des Schlittens 7 erstreckt. Auf mittlerer Höhe der Nut 11 ist eine Ausnehmung 12 mit kreisförmigem Querschnitt eingeformt, die in Fig. 2 als Durchbruch bzw. Bohrung 12 ausgestaltet ist. In Querrichtung erstreckt sich die Nut 11 nicht über den gesamten Querschnitt des Schlittens 7, sondern über etwa einem Drittel des Querschnitts des Schlittens 7. Jenseits der beiden Nutränder 13 ist ein jeweiliger Endbereich 14 des Schlittens 7 abgeschrägt eingeformt. Auf einer der Nut 11 abgewandten Stirnseite 15 des Schlittens 7 weist der Schlitten 7 im Wesentlichen einen rechteckigen Querschnitt mit abgerundeten Ecken auf. Auf mittlerer Länge jeweils einer Seitenkante der Stirnseite 15 ist ein rechteckförmiger Schlitz 16 in eine zugeordnete Mantelfläche 17 des Schlittens 7 eingearbeitet, der sich in etwa über die halbe Höhe des Schlittens 7 erstreckt.

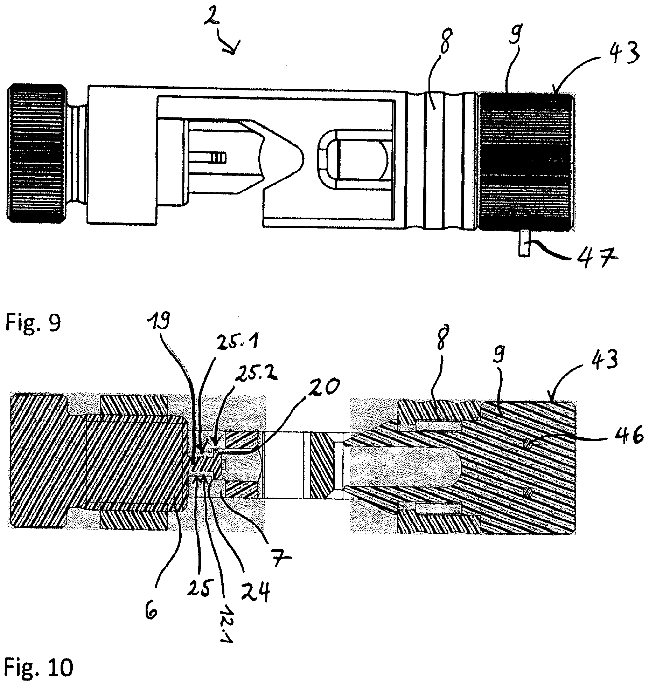

Der Schlitten 7 weist von seiner Stirnseite 15 aus zentral eine Bohrung 12.1 auf, die sich etwa über die Höhe der Schlitze 16 erstreckt, mit der Bohrung 12 fluchtet und in diese übergeht. Die Bohrung 12.1 weist aber einen geringeren Durchmesser auf als die Bohrung 12, so dass beim Übergang beider Bohrungen 12, 12.1 eine Hinterschneidung 24 gebildet wird, was detailliert aus Fig. 10 hervorgeht.

Fig. 3 zeigt das Schraubteil 6 des Anpressstücks 4, wobei das Schraubteil 6 eine im Wesentlichen zylindrische Form aufweist. An einer dem Schlitten 7 zugewandten Stirnseite 18 weist das Schraubteil 6 einen radial zentrierten, zylinderförmigen Zapfen 19 auf, der, dem Schlitten 7 zugewandt, mit einem Ansatz 20 versehen ist. Der Ansatz 20 ist kegelstumpfförmig ausgebildet und weist einen größeren Durchmesser auf als der Zapfen 19. Der Ansatz 20 weist einen Durchmesser größer als der der Bohrung 12.1 auf (während der Durchmesser des Zapfens 19 kleiner als der der Bohrung 12.1 ist und kleiner als der der Bohrung 12). Auf einer dem Zapfen 19 abgewandten Seite ist das Schraubteil 6 mit einem sockelförmigen Endstück 21 versehen, in dessen Mantelfläche eine Rändelung 22 eingearbeitet ist. Fig. 4 zeigt das Schraubteil 6 aus Fig. 3 in einer Seitenansicht. Die Mantelfläche 23 des Schraubteils 6 weist ein Außengewinde auf, das in den Fig. 3 und 4 nicht dargestellt ist.

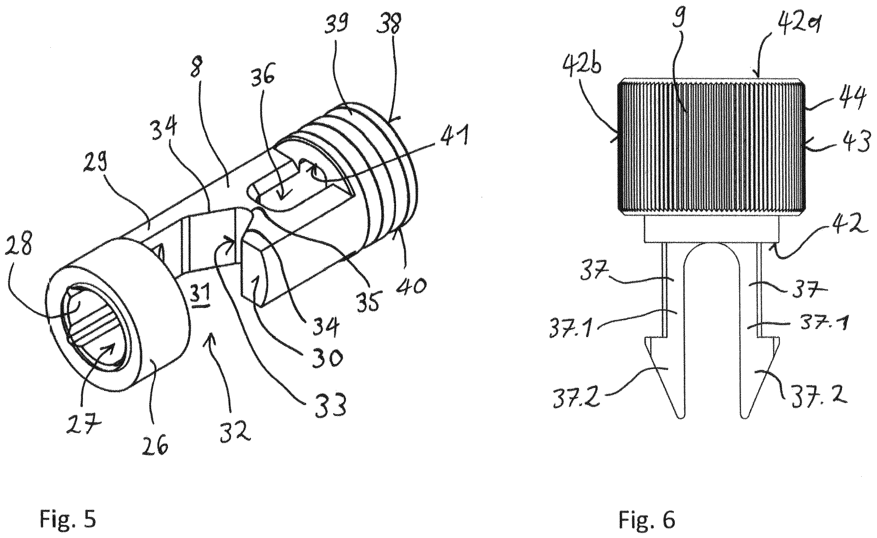

In Fig. 5 ist das Halteteil 8 des Gegenstücks 3 dargestellt, das in einem dem Anpressstück 4 zugewandten Bereich einen hohlzylindrischen Ansatz 26 mit einem zentrierten Durchbruch 27 aufweist. Eine Innenwandung 28 des Durchbruchs 27 ist mit einem Innengewinde (nicht dargestellt) versehen, so dass der Durchbruch 27 als Gewindebohrung ausgestaltet ist. Das Gewinde der Innenwand 28 entspricht dem Gewinde des Schraubteils 6. Auf der dem Schraubteil 6 abgewandten Seite des Ansatzes 26 ist ein bogenförmiger Haltebereich 29 eingeformt, der in einer zum Ansatz 26 weisenden Stirnseite 30 endet und einen inneren freien Aufnahmebereich 31 teilweise rahmenartig umgibt. Zwischen der Stirnseite 30 und dem Ansatz 26 gibt ein Durchbruch 32 den Aufnahmebereich 31 frei.

In einem dem Ansatz 26 gegenüberliegenden Abschnitt weist der Haltebereich 29 eine Vertiefung 33 auf, mit ersten gegenüberliegenden, gerade und winklig zueinander verlaufenden Abschnitten 34 und einem zweiten, zentriert angeordneten und gekrümmten Endabschnitt 35, der die beiden Abschnitte 34 verbindet.

In einem dem Ansatz 26 abgewandten Endbereich des Haltebereichs 29 sind zwei einander gegenüberliegende, rechteckförmige und als Durchbrüche ausgestaltete Ausnehmungen 36 zur Aufnahme von Rasthaken 37 des Steckers 9 (Fig. 6) eingeformt. Auf der dem Ansatz 26 gegenüber liegenden Stirnseite 38 ist ein hohlzylinderförmiges Endstück 39 mit einer profilierten Mantelfläche 40 einstückig mit dem Haltebereich 29 verbunden. In der radialen Innenfläche des Endstücks 39 sind Ausnehmungen 41 eingeformt, mit denen die Rasthaken 37 in Eingriff bringbar sind und die sich an die Durchbrüche 36 anschließen.

Fig. 6 zeigt den Stecker 9 in einer Seitenansicht. Auf einer dem Halteteil 8 zugewandten Stirnseite 42 sind die zwei sich in Längsrichtung erstreckenden und einander gegenüber liegenden Rasthaken 37 mit jeweils einem axial verlaufenden Schenkel 37.1 und einem hakenartigen Vorsprung 37.2 ausgebildet, die mit den Durchbrüchen 36 und den Ausnehmungen 41 des Halteteils 8 in Eingriff bringbar sind. Auf diese Weise wird der Stecker 9 mit dem Halteteil 8 verbunden. Auf einer den Rasthaken 37 abgewandten Stirnseite 42a weist der Stecker 9 ein zylinderförmiges Endstück 42b auf, dessen Mantelfläche 43 mit einer Rändelung 44 versehen ist. Die Rändelung 44 dient, ähnlich wie die Rändelung 22 des Schraubteils 6, zur einfachen manuellen Betätigung der Einrichtung 1.

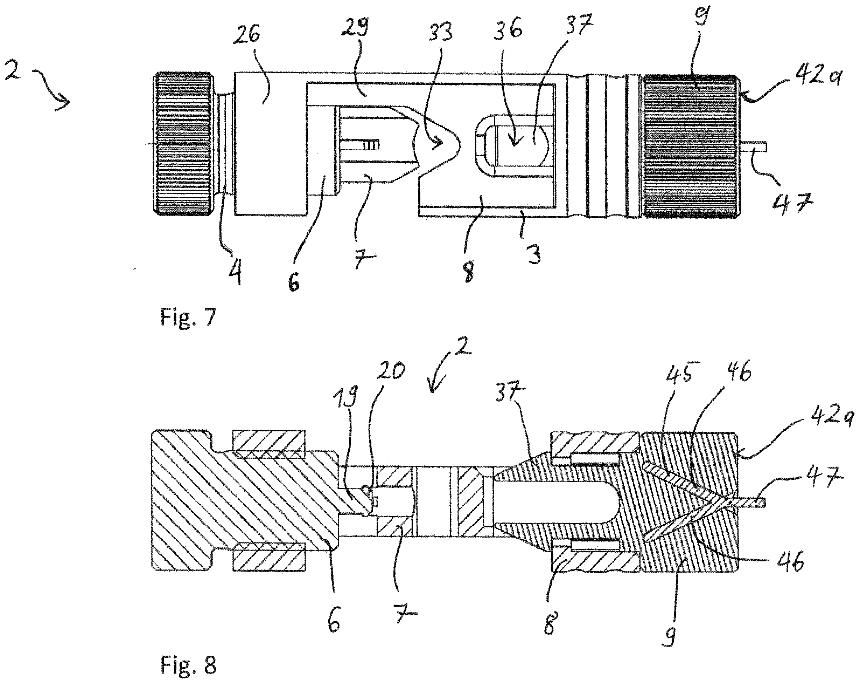

In Fig. 7 ist die Befestigungsvorrichtung 1 als zusammengesetztes Spannelement 2 gezeigt, wobei das Schraubteil 6 des Anpressstücks 4 mittels einer Schraubbewegung durch den Ansatz 26 des Halteteils 8 geführt ist. Zur Befestigung des Schraubteils 6 mit dem Schlitten 7 wird das Schraubteil 6 von der Stirnseite 15 des Schlittens 7 her mit dem Zapfen 19 und dem Ansatz 20 in die Bohrung 12.1 des Schlittens 7 eingedrückt. Aufgrund der Schlitze 16 können die zwischen diesen verbleibenden Materialbereiche zurückweichen, so dass der Ansatz 20 trotz gegenüber dem Durchmesser der Bohrung 12.1 größeren Durchmessers durch die Bohrung 12.1 hindurch gedrückt werden kann bis der Ansatz 20 in die Bohrung 12 eintritt und die Hinterschneidung 24 am Übergang der Bohrungen 12, 12.1 hintergreift, wie dies in Fig. 8 und 10 dargestellt ist. Dort sind Schraubteil 6 und Schlitten 7 untrennbar verbunden, allerdings relativ zueinander drehbar, wobei der Schlitten 7 jedoch aufgrund des Haltebereichs 29 des Halteteils 8 an einer vollumfänglichen Drehbewegung gehindert ist. Mittels der Schraubbewegung ist das Schraubteil 6 und damit das Anpressstück 4 axial beweglich relativ zum Gegenstück 3. Insbesondere ist der Schlitten 7 auf diese Weise in Richtung der Vertiefung 33 des Halteteils 8 beweglich.

In dem in Fig. 7 rechts dargestellten, dem Schlitten 7 abgewandten Bereich ist die Verbindung des Steckers 9 mit dem Halteteil 8 ersichtlich. Wie oben bereits erwähnt, durchgreifen die Rasthaken 37 des Steckers 9 die Ausnehmungen 41 und rasten in Durchbrüchen 36 des Halteteils 8 ein, so dass der Stecker 9 mit dem Halteteil 8 formschlüssig und lösbar verbunden ist. Zum Lösen der Verbindung werden die Rasthaken 37 vom Benutzer manuell radial nach innen gedrückt und der Stecker 9 daraufhin vom Halteteil 8 entfernt.

Ein elektromagnetischer Sensor 45 ist in dem in Fig. 7 gezeigten Ausführungsbeispiel im Stecker 9 vorgesehen, was insbesondere aus der zur Fig. 7 zugehörigen Längsschnittzeichnung der Fig. 8 hervorgeht. Dort ist ersichtlich, dass der Sensor 45 zwei spiralförmig gewendelte Spulen 46 aus beispielsweise durch Isolierlack elektrisch isoliertem Metalldraht aufweist, wobei insbesondere der Metalldraht von Isolierlack ummantelt ist, und in den Stecker 9 eingeklebt ist. Eine an den Sensor 45 angeschlossene Sensorleitung 47 tritt an der dem Anpressstück 4 abgewandten Stirnseite 42a aus dem Stecker 9 aus. Die Spulen 46 stehen in V-Stellung zueinander und bilden so einen 6-DOF-Sensor.

Die Fig. 9 und 10 zeigen eine zweite Ausführungsform des Spannelements 2, bei dem der Sensor 45 in einer senkrecht zur Längsschnittebene der Fig. 10 gezeigten Anordnung im Stecker 9 vorgesehen ist. In dieser Ausführungsform tritt die Sensorleitung 47 aus der Mantelfläche 43 des Steckers 9 aus. Zwischen dem Zapfen 19 des Schraubteils 6 und einer Innenwandung 25 der Ausnehmung 12 ist ein radialer Zwischenraum oder radiales Spiel 25.1 ausgebildet. Außer dem radialen Spiel 25.1 zwischen dem Zapfen 19 und der Innenwandung 25 ist zwischen dem Ansatz 20 und der Hinterschneidung 24 ein axiales Spiel 25.2 ausgestaltet, so dass relative Verkippungen zwischen dem Schraubteil 6 und dem Schlitten 7, wobei beide Achsen außer Flucht gebracht werden, leichter aufgenommen werden können.

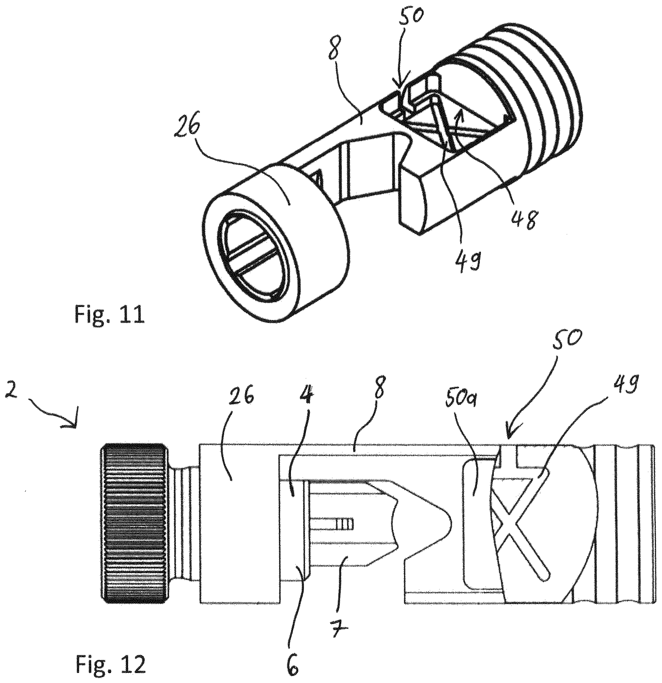

Fig. 11 zeigt eine weitere Ausgestaltung des Halteteils 8, das in einem dem Ansatz 26 abgewandten Bereich eine rechteckförmige Vertiefung 48 mit abgerundeten Ecken aufweist, auf deren Boden eine kreuzförmige Nut 49 zur Einbringung eines entsprechenden Sensors 45 eingearbeitet ist, so dass die Nut 49 als Sensoraufnahme dient. In dieser Ausgestaltung können die Sensorleitungen 47 durch einen seitlichen Durchbruch 50 aus dem Halteteil 8 herausgeführt werden. Fig. 12 zeigt den Aufbau des Halteteils 8 aus Fig. 11 als Teil einer weiteren Ausführungsform der Einrichtung 2 in einer schematischen Aufsicht, bei der der Bereich der Vertiefung 48 teilweise als schematischer Längsschnitt gezeichnet ist, um die Lagen der Fräsung 49 und des Durchbruchs 50 zu veranschaulichen. Die Vertiefung 48 kann mit einer Abdeckung 50a lösbar verschlossen, insbesondere verklebt werden, um den Sensor 45 bzw. die Sensoraufnahme 49 vor Verschmutzungen zu schützen. Alternativ kann die Abdeckung 50a mit der Vertiefung 48 verschweißt werden. Gemäß Fig. 12 ist das Anpressstück 4 aus dem Schraubteil 6 und dem Schlitten 7 mit dem Halteteil 8 verbunden.

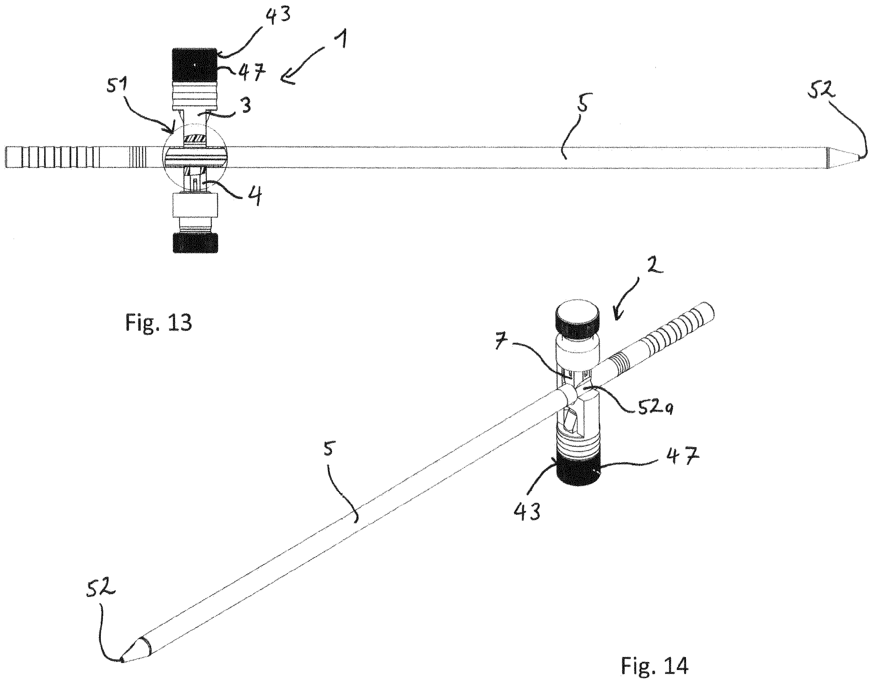

Fig. 13 zeigt die erfindungsgemäße medizinische Befestigungseinrichtung 1, bei der ein längliches medizinisches Instrument 5 zwischen dem Anpressstück 4 und dem Gegenstück 3 eingeklemmt ist. Der Verbindungsbereich 51 ist der besseren Übersichtlichkeit wegen im Längsschnitt dargestellt. Durch die in Fig. 13 gezeigte Anordnung nimmt der im Stecker 9 angeordnete Sensor 45 eine in Bezug auf eine distale Spitze 52 des Instruments 5 stets feste Relativposition ein. Wird anhand gängiger Methoden die Position des Sensors 9 beispielsweise innerhalb eines extern angelegten elektromagnetischen Feldes bestimmt, kann aus diesen Informationen auf die räumliche Position und Ausrichtung der distalen Spitze 52 des Instruments 5 geschlossen werden.

In Fig. 14 ist die Anordnung aus Fig. 13 in einer perspektivischen Ansicht dargestellt, aus der hervorgeht, dass das Instrument 5 im Verbindungsbereich 51 mit einer als Ringnut ausgestalteten Vertiefung 52a versehen ist, deren Länge der Länge der Nut 11 des Schlittens 7 entspricht. Auf diese Weise ist die axiale Längsposition der Einrichtung 1 am Instrument 5 bestimmbar. In den Fig. 13 und 14 ist der Austritt der Sensorleitung 47 aus der Mantelfläche 43 des Steckers 9 gezeigt.

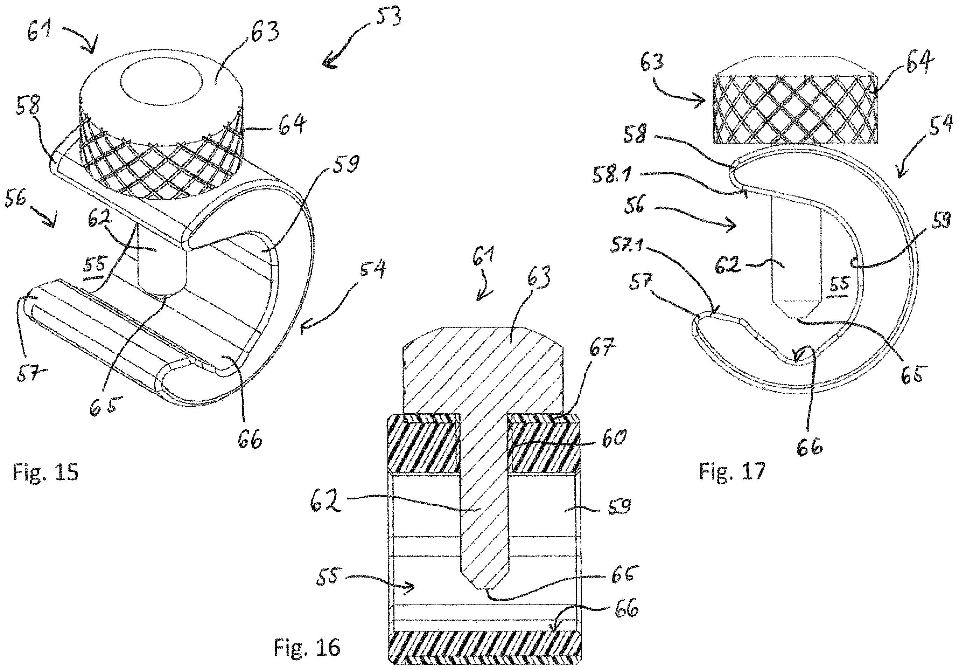

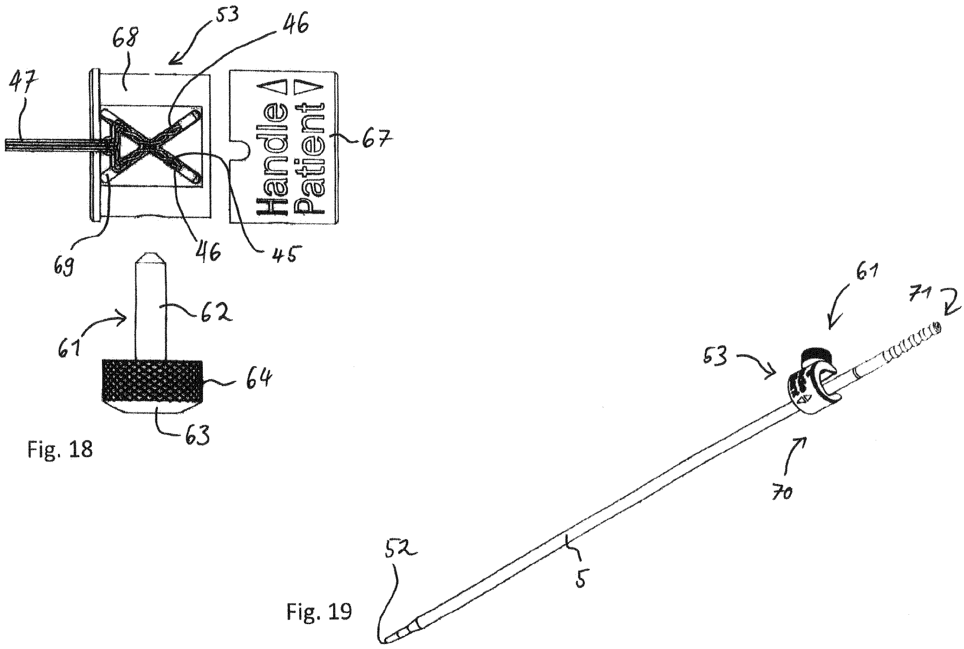

Die Fig. 15 bis 18 zeigen eine weitere Ausgestaltung der erfindungsgemäßen Befestigungseinrichtung und die Fig. 19 bis 22 eine erfindungsgemäße medizinische Vorrichtung mit einer solchen Einrichtung.

Die erfindungsgemäße Einrichtung 53 der Fig. 15 bis 18 umschließt mit einem teilkreisförmigen Bügel 54 einen Innenraum 55 und weist ebenfalls eine seitliche Öffnung 56 auf, wie die vorigen Einrichtungen 1. Der Abstand der freien Stirnseiten des Bügels 54 bzw. die Größe der Öffnung 56 ist geringer als der Durchmesser des umschlossenen Innenraums 55 (Fig. 17). Bei dieser Ausgestaltung sind die Endbereiche von Teilbögen, die den der Öffnung 56 zugewandten freien Stirnseiten 57.1, 58.1 der Einzelwangen 57, 58 abgewandt sind, einstückig miteinander verbunden bzw. gehen einstückig ineinander über, so dass die Wangen hier einen starren einstückigen teilringförmigen Bügel 54 mit inneren Längswandungen 59 bilden. Dieser weist nahe der Stirnseite 58.1 eine radiale Gewindebohrung 60 auf, in die eine Schraube 61 mit einem mit einem Außengewinde versehenen Schraubenschaft 62 und einem Schraubenkopf 63 mit einer Umfangsrändelung 64 eingeschraubt ist. Dem inneren freien Ende 65 des Schraubenschafts 61 gegenüberliegend ist die Innenseite des Bügels 54 mit einer inneren Aufnahme 66 versehen, die z.B. prismenförmig ausgeführt sein kann und ähnlich zur Vertiefung 33 der vorigen Ausführungen ausgestaltet ist.

Die Einrichtung 53 weist unter einer axial aufschiebbaren Abdeckung 67 in einem Innenteil 68 des Bügels 54 eine Aufnahme 69 in Form einer kreuzförmigen Vertiefung auf, in die der Sensor 45 eingeklebt wird, welcher über die Sensorleitungen 47 mit einer Auswerteeinheit verbindbar ist.

Die Einrichtung 53 kann seitlich oder radial über ein chirurgisches Instrument, Werkzeug oder chirurgisches Teil 5 auf dieses aufgebracht werden (Fig. 19 bis 22), indem die Schraube 61 zurückgeschraubt ist, so dass der Innenraum 55 des Bügels 54 freigegeben ist und beim Aufbringen der Einrichtung 53 auf das chirurgische Instrument 5 dieses in den Innenraum 55 eintreten kann. Anschließend wird die Schraube nach innen geschraubt, wodurch das chirurgische Instrument 5 in die Aufnahme 66 eingedrückt wird und bei weiterem Schrauben die Einrichtung 53 an dem chirurgischen Instrument 5 festgelegt wird. Ein langer Gewindegang der Schraube und die Ausgestaltung der Längswandungen 59 der Einrichtung 53 ermöglichen die Verwendung für einen großen Durchmesserbereich.

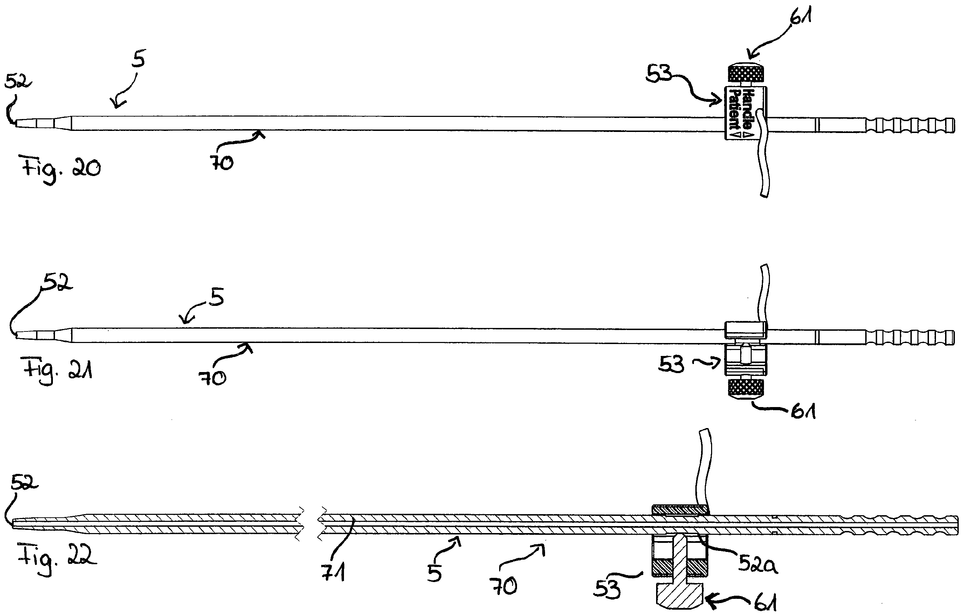

Gemäß der Figuren 19 bis 22 ist eine bevorzugte Ausgestaltung der Gesamt-Sensorvorrichtung 70 durch ein chirurgisches Instrument, Werkzeug oder chirurgisches Teil 5 und einer an diesem festgelegten Einrichtung 53, wie sie vorstehend beschrieben wurde, gebildet. Auch hier weist das chirurgische Instrument 5 eine Umfangsnut 52a auf, wodurch die Einrichtung 53 am Instrument 5 axial definiert festgelegt bzw. positioniert wird, so dass bei Messung der Einrichtung 53 bzw. über den in ihr befindlichen Sensor 45 wieder die genaue Position der distalen Spitze 52 des chirurgischen Instruments 5 bestimmbar ist. Das chirurgische Instrument 5 kann, wie in Fig. 22 dargestellt, kanuliert sein, also einen Axialdurchbruch 71 aufweisen, wie er in der Schnittdarstellung der Fig. 22 erkennbar ist, so dass die Vorrichtung 70 über einen Draht, wie über einen Seldinger-Draht, in einen Patienten eingeführt werden kann.

## Patentansprüche

1. Medizinische Befestigungseinrichtung (1) für einen Sensor (45) mit einem Spannelement (2) und einer Sensoraufnahme (49), wobei das Spannelement (2) ein Gegenstück (3) und ein relativ zum Gegenstück (3) bewegliches Anpressstück (4) aufweist, wobei das Gegenstück (3) einen seitlichen Durchbruch (32) aufweist, der einen freien Aufnahmebereich (31) freigibt, wobei das Gegenstück (3) den Aufnahmebereich (31) zumindest teilweise rahmenartig umgibt, wobei das Gegenstück (3) eine zum Aufnahmebereich (31) weisende Vertiefung (33) aufweist, die dem Anpressstück (4) gegenüber angeordnet ist, wobei das Gegenstück (3) mit einem der Vertiefung (33) gegenüber angeordneten Durchbruch (27) versehen ist und wobei das Anpressstück (4) in dem Durchbruch (27) in Richtung auf die Vertiefung (33) des Gegenstücks (3) zu bewegbar ist, wobei die Befestigungseinrichtung zur Befestigung eines elektromagnetischen Sensors ausgebildet ist, eine Sensoraufnahme (49) vorgesehen ist, das Anpressstück (4) einen Schlitten (7) und ein Schraubteil (6) aufweist, **dadurch gekennzeichnet, dass** der Schlitten (7) mit dem Schraubteil (6) formschlüssig verbunden und an diesem drehbar gelagert ist und dass weiterhin der Schlitten (7) an seiner der Vertiefung (33) des Gegenstücks (3) zugewandten Stirnseite (19) eine im Schnitt kreissegmentförmige Nut (11) aufweist.

2. Einrichtung nach 1, **dadurch gekennzeichnet, dass** die Vertiefung (33) erste, gegenüber liegende, gerade, winklig zueinander verlaufende Abschnitte (34) und einem zweiten, gekrümmten Endabschnitt (35), der die ersten Abschnitte (34) verbindet, aufweist.

3. Einrichtung nach einem der Ansprüche 1 2-bis 2, **dadurch gekennzeichnet, dass** das Gegenstück (3) als einstückiger, umfangsseitig teilweise offener Bügel (54) ausgestaltet ist mit zwei Wangen (57, 58), die einander zugewandte Stirnseiten (57.1, 58.1) ausbilden und einen endlichen Abstand zueinander aufweisen.

4. Einrichtung nach einem der vorangehenden Ansprüche, **dadurch gekennzeichnet, dass** das Schraubteil (6) an einer dem Schlitten (7) zugewandten Stirnseite (18) einen Zapfen (19) aufweist, der in eine Ausnehmung (12) des Schlittens (7) in Eingriff bringbar ist, insbesondere der Zapfen (19) einen dem Schlitten (7) zugewandten Ansatz (20) aufweist, dessen Querschnitt größer ist als der Querschnitt des Zapfens (19), dass der Schlitten (7) an seiner, dem Schraubteil (6) zugewandten Stirnseite (15) eine Bohrung (12.1) aufweist, deren Durchmesser kleiner ist als der Durchmesser der Ausnehmung (12), so dass zwischen der Bohrung (12.1) und der Ausnehmung (12) eine Hinterschneidung (24) ausgebildet ist und dass der Ansatz (20) die Hinterschneidung (24) hintergreift.

5. Einrichtung nach Anspruch 4, **dadurch gekennzeichnet, dass** der Zapfen (19) des Schraubteils (6) innerhalb der Ausnehmung (12) des Schlittens (7) derart radial beabstandet zur Innenwandung (25) der Ausnehmung (12) angeordnet ist, dass zwischen dem Zapfen (19) und der Ausnehmung (12) ein radiales Spiel (25.1) ausgebildet ist.

6. Einrichtung nach einem der Ansprüche 4 oder 5, **dadurch gekennzeichnet, dass** der Ansatz (20) derart axial beabstandet zur Hinterschneidung (24) angeordnet ist, dass zwischen dem Ansatz (20) und der Hinterschneidung (24) ein axiales Spiel (25.2) ausgebildet ist.

7. Einrichtung nach einem der vorangehenden Ansprüche, **dadurch gekennzeichnet, dass** das Gegenstück (3) einen Stecker (9) und ein mit dem Stecker formschlüssig verbundenes Halteteil (8) aufweist, wobei insbesondere der Stecker (9) mit dem Halteteil (8) lösbar verbunden ist.

8. Einrichtung nach Anspruch 7, **dadurch gekennzeichnet, dass** der Stecker (9) mit dem Halteteil (8) mittels einer Rast- und/oder einer Schnappverbindung verbunden ist, wobei insbesondere der Stecker (9) an seiner dem Halteteil (8) zugewandten Stirnseite (42) mindestens zwei sich in axialer Richtung erstreckende Rasthaken (37) mit jeweils einem Schenkel (37.1) und einem hakenartigen Vorsprung (37.2) aufweist, die mit jeweils einer Ausnehmung (36) des Halteteils (8) in Eingriff bringbar sind.

9. Einrichtung nach Anspruch 7 oder 8, **dadurch gekennzeichnet, dass** der Stecker (9) an seinem dem Halteteil (8) und/oder den Schenkeln (37.1) abgewandten Ende ein zylindrisches Endstück (42b) mit einer Umfangsrändelung (44) aufweist.

10. Einrichtung nach einem der vorangehenden Ansprüche, **dadurch gekennzeichnet, dass** die Sensoraufnahme (49) im Gegenstück (3), insbesondere in dessen Stecker (9), angeordnet ist.

11. Einrichtung nach einem der vorangehenden Ansprüche, **dadurch gekennzeichnet, dass** die Sensoraufnahme (49) kreuzförmig oder V-förmig ausgestaltet ist, wobei insbesondere die Sensoraufnahme (49) mit einer Abdeckung (50a, 67) versehen ist.

12. Einrichtung nach einem der vorangehenden Ansprüche, **dadurch gekennzeichnet, dass** die Einrichtung (1) einen elektromagnetischen Sensor (45) aufweist, der als mindestens zwei unter endlichem Winkel zueinander angeordnete, spiralförmig gewendelte Spulen (46) ausgestaltet ist.

13. Medizinische Vorrichtung mit einer Befestigungseinrichtung (1) nach einem der Ansprüche 1 bis 12, einem elektromagnetischen Sensor (45) und einem medizinischem Instrument (5), **dadurch gekennzeichnet, dass** das Instrument (5) in den Aufnahmebereich (31) des Gegenstücks (3) einführbar ist, wobei die Befestigungseinrichtung (1) mit dem Instrument (5) klemmend lösbar verbunden ist und wobei der Sensor (45) in der Sensoraufnahme (49) angeordnet ist, wobei insbesondere das Instrument (5) mit einer Umfangsnut (52a) versehen ist und dass die Breite des Schlittens (7) der axialen Länge der Umfangsnut (52a) des Instruments (5) entspricht.

14. Vorrichtung nach Anspruch 13, **dadurch gekennzeichnet, dass** das Instrument (5) im Bereich der Umfangsnut (52a) flächig auf dem zweiten Endabschnitt (35) der Vertiefung (33) des Halteteils (8) und/oder auf der Nut (11) des Schlittens (7) aufliegt.

## Claims

1. Medical fastening device (1) for a sensor (45) with a clamping element (2) and with a sensor mount (49), wherein the clamping element (2) has a counterpiece (3) and a pressing piece (4) movable relative to the counterpiece (3), wherein the counterpiece (3) has a lateral opening (32), which frees a free mounting area (31), wherein the counterpiece (3) encloses the mounting area (31) at least partially in a framelike manner, wherein the counterpiece (3) has a depression (33), which points towards the mounting area (31) and which is arranged opposite the pressing piece (4), wherein the counterpiece (3) is provided with an opening (27) arranged opposite the depression (33), and wherein the pressing piece (4) is movable in the opening (27) in the direction of the depression (33) of the counterpiece (3), wherein the fastening device is configured for receiving an electromagnetic sensor, wherein a sensor mount (49) is provided, the pressing piece (4) has a carriage (7) and a screwed part (6), **characterized in that** the carriage (7) is connected to the screwed part (6) in a positive-locking manner and is mounted rotatably at the screwed part (6) and that moreover, the carriage (7) has a groove (11) having a circle segment-shaped section on its end face (19) facing the depression (33) of the counterpiece (3).

2. Device according to claim 1, **characterized in that** the depression (33) has first, opposite, straight sections (34) extending at an angle in relation to one another and a second, curved end section (35), which connects the first sections (34).

3. Device in accordance with one of the claims 1 or 2, **characterized in that** the counterpiece (3) is configured as a one-piece, circumferentially partially open strap (54) with two cheeks (57, 58), which form end faces (57.1, 58.1) facing each other and have a finite distance from one another.

4. Device in accordance with one of the preceding claims, **characterized in that** on an end face (18) facing the carriage (7), the screwed part (6) has a pin (19), which can be caused to mesh with a recess (12) of the carriage (7), in particular that the pin (19) has an attachment (20), which faces the carriage (7) and whose cross section is larger than the cross section of the pin (19), that the carriage (7) has, on its end face (15) facing the screwed part (6), a hole (12.1), whose diameter is smaller than the diameter of the recess (12), so that an undercut is (24) formed between the hole (12.1) and the recess (12), and that the attachment (20) extends behind the undercut (24).

5. Device in accordance with claim 4, **characterized in that** the pin (19) of the screwed part (6) is arranged within the recess (12) of the carriage (7) at a radially spaced location from the inner wall (25) of the recess (12) such that a radial clearance (25.1) is formed between the pin (19) and the recess (12).

6. Device in accordance with one of the claims 4 or 5, **characterized in that** the attachment (20) is arranged at an axially spaced location from the undercut (24) such that an axial clearance (25.2) is formed between the attachment (20) and the undercut (24).

7. Device in accordance with one of the preceding claims, **characterized in that** the counterpiece (3) has a plug (9) and a holding part (8) connected to the plug in a positive-locking manner, wherein in particular the the plug (9) is detachably connected to the holding part (8).

8. Device in accordance with claim 7, **characterized in that** the plug (9) is connected to the holding part (8) by means of a locking connection and/or snap connection, wherein in particular the plug (9) has, on its end face (42) facing the holding part (8), at least two locking hooks (37) extending in the axial direction with a respective leg (37.1) and with a respective hook-like projection (37.2), which can be caused to mesh with a recess (36) of the holding part (8).

9. Device in accordance with one of the claims 7 or 8, **characterized in that** the plug (9) has, at its end facing away from the holding part (8) and/or the legs (37.1), a cylindrical end piece (42b) with a circumferential knurling (44).

10. Device in accordance with one of the preceding claims, **characterized in that** the sensor mount (49) is arranged in the counterpiece (3), especially in the plug (9) thereof.

11. Device in accordance with one of the preceding claims, **characterized in that** the sensor mount (49) has a cross-shaped or V-shaped configuration, wherein in particular the sensor mount (49) is provided with a cover (50a, 67).

12. Device in accordance with one of the preceding claims, **characterized in that** the device (1) has an electromagnetic sensor (45), which is configured as at least two helically wound coils (46) arranged at a finite angle in relation to one another.

13. Medical device with a fastening device (1) in accordance with one of the claims 1 to 12, with an electromagnetic sensor (45) and with a medical instrument (5), **characterized in that** the instrument (5) can be inserted into the mounting area (31) of the counterpiece (3), wherein the fastening device (1) can be connected by clamping to the instrument (5) in a detachable manner, and wherein the sensor (45) is arranged in the mount (49), wherein in particular the instrument (5) is provided with a circumferential groove (52a) and that the width of the carriage (7) corresponds to the axial length of the circumferential groove (52a) of the instrument (5).

14. Device according to claim 13, **characterized in that** the instrument (5) lies in the area of the circumferential groove (52a) flatly on the second end section (35) of the depression (33) of the holding part (8) and/or on the groove (11) of the carriage (7).

## Revendications

1. Appareil médical de fixation (1) pour un capteur (45) avec un élément de serrage (2) et un logement de capteur (49), l'élément de serrage (2) comportant un pendant (3) et une partie de compression (4) mobile par rapport au pendant (3), le pendant (3) comportant un passage traversant latéral (32) qui libère une zone de logement libre (31), le pendant (3) entourant au moins en partie à la façon d'un cadre la zone de logement (31), le pendant (3) comportant un renforcement (33) orienté vers la zone de logement (31), ledit renfoncement étant disposé en vis-à-vis par rapport à la partie de compression (4), le pendant (3) étant pourvu d'un passage traversant (27) disposé en vis-à-vis par rapport au renforcement (33) et la partie de compression (4) située dans le passage traversant (27) pouvant être déplacée en direction du renforcement (33) du pendant (3), l'appareil de fixation étant réalisé pour fixer un capteur électromagnétique, un logement de capteur (49) étant prévu, la partie de compression (4) comportant un coulisseau (7) et une partie vissée (6), **caractérisé en ce que** le coulisseau (7) est relié à la partie vissée (6) par complémentarité de formes et est disposé de façon à pouvoir tourner au niveau de celle-ci et qu'en outre le coulisseau (7) comporte une rainure (11) en forme de segment de cercle au niveau de son côté avant (19) orienté vers le renforcement (33) du pendant (3).

2. Appareil selon la revendication 1, **caractérisé en ce que** le renforcement (33) comporte des premières sections (34) opposées droites s'étendant en angle les unes par rapport aux autres et une deuxième section d'extrémité (35) incurvée reliant entre elles les premières sections (34).

3. Appareil selon l'une quelconque des revendications 1 à 2, **caractérisé en ce que** le pendant (3) est réalisé sous la forme d'un étrier (54) d'un seul tenant en partie ouvert sur le côté de sa périphérie avec deux joues (57, 58) formant des côtés avant (57.1, 58.1) orientés l'un vers l'autre et une distance finie l'une par rapport à l'autre.

4. Appareil selon l'une quelconque des revendications précédentes, **caractérisé en ce que** la partie vissée (6) comporte un tenon (19) au niveau d'un côté avant (18) orienté vers le coulisseau (7), ledit tenon pouvant être amené en prise dans un évidement (12) du coulisseau (7), le tenon (19) comportant notamment un appendice (20) orienté vers le coulisseau (7) dont la section transversale est plus importante que la section transversale du tenon (19), que le coulisseau (7) comporte au niveau de son côté avant (15) orienté vers la partie vissée (6) un alésage (12.1) dont le diamètre est inférieur au diamètre de l'évidement (12), de sorte qu'un détouré arrière (24) est réalisé entre l'alésage (12.1) et l'évidement (12) et que l'appendice (20) engrène par l'arrière le détouré arrière (24).

5. Appareil selon la revendication 4, **caractérisé en ce que** le tenon (19) de la partie vissée (6) est disposé de telle sorte à l'intérieur de l'évidement (12) du coulisseau (7), à une certaine distance dans le plan radial par rapport à la paroi intérieure (25) de l'évidement (12), qu'un jeu radial (25.1) est obtenu entre le tenon (19) et l'évidement (12).

6. Appareil selon l'une quelconque des revendications 4 ou 5, **caractérisé en ce que** l'appendice (20) est disposé de telle sorte dans le plan axial à une certaine distance du détouré arrière (24) qu'un jeu axial (25.2) est obtenu entre l'appendice (20) et le détouré arrière (24).

7. Appareil selon l'une quelconque des revendications précédentes, **caractérisé en ce que** le pendant (3) comporte un connecteur (9) et une partie de maintien (8) reliée par complémentarité de formes au connecteur, le connecteur (9) étant notamment relié de façon amovible à la partie de maintien (8).

8. Appareil selon la revendication 7, **caractérisé en ce que** le connecteur (9) est relié à la partie de maintien (8) au moyen d'une liaison par arrêt et/ou encliquetage, le connecteur (9) comportant notamment au niveau de son côté avant (42) orienté vers la partie de maintien (8) au moins deux crochets d'arrêt (37) s'étendant dans la direction axiale avec respectivement un flanc (37.1) et une saillie (37.2) de type crochet pouvant respectivement être amenés en prise avec respectivement un évidement (36) de la partie de maintien (8).

9. Appareil selon la revendication 7 ou 8, **caractérisé en ce que** le connecteur (9) comporte au niveau de son extrémité opposée à la partie de maintien (8) et/ou au flanc (37.1) une partie d'extrémité (42b) cylindrique avec un moletage périphérique (44).

10. Appareil selon l'une quelconque des revendications précédentes, **caractérisé en ce que** le logement de capteur (49) est disposé dans le pendant (3), notamment dans son connecteur (9).

11. Appareil selon l'une quelconque des revendications précédentes, **caractérisé en ce que** le logement de capteur (49) est configuré en forme de croix ou en forme de V, le logement de capteur (49) étant notamment pourvu d'un cache (50a, 67).

12. Appareil selon l'une quelconque des revendications précédentes, **caractérisé en ce que** l'appareil (1) comporte un capteur (45) électromagnétique qui est configuré sous la forme d'au moins deux bobines (46) en forme de spirale disposées selon un certain angle l'une par rapport à l'autre.

13. Appareil médical équipé d'un appareil de fixation (1) selon l'une quelconque des revendications 1 à 12, d'un capteur électromagnétique (45) et d'un instrument médical (5), **caractérisé en ce que** l'instrument (5) peut être introduit dans la zone de logement (31) du pendant (3), l'appareil de fixation (1) étant relié de façon amovible et par serrage à l'instrument (5) et le capteur (45) étant disposé dans le logement de capteur (49), l'instrument (5) étant notamment pourvu d'une rainure périphérique (52a) et que la largeur du coulisseau (7) correspond à la longueur axiale de la rainure périphérique (52a) de l'instrument (5).

14. Appareil selon la revendication 13, **caractérisé en ce que** l'instrument (5) reposant à plat dans la région de la rainure périphérique (52a) sur la deuxième section d'extrémité (35) du renforcement (33) de la partie de maintien (8) et/ou sur la rainure (11) du coulisseau (7).
